# EUROPEAN PATENT APPLICATION

(11) **EP 2 942 075 A1**
(43) Date of publication of application: **11.11.2015**
(21) Application number: 14167428.3
(22) Date of filing: 07.05.2014
(51) Int. Cl.: A61M 16/20, A61M 16/08

(54) **Device and method for a low resistance valve**

(71) Applicant: Mindray Medical Sweden AB, 174 57 Sundbyberg (SE)
(72) Inventor: Laksov, Joakim, 182 56 Danderyd (SE); Ringden, Ola, 172 37 Sundbyberg (SE)
(74) Representative: KIPA AB

(57) **Abstract**

A valve (1, 2, 3, 4) for a respiratory device is disclosed. The valve includes a valve housing (10, 20, 30 ,40) having an inlet (11, 21, 31, 41) and an outlet (12, 22, 32, 42). The valve further includes a tubular flow channel (18, 28) arranged in the valve housing and thereby forming a chamber (15, 25) between an inner surface of the valve housing and an outer surface (16, 26) of the tubular flow channel. One end of the tubular flow channel is the inlet and an edge at the other end of the tubular flow channel is configured as a valve seat (17, 27). In this way, a fluid flow from the inlet flows through the tubular flow channel and over the valve seat into the chamber. The outlet has an outlet housing and the outlet is arranged perpendicular to the inlet. The outlet of the valve has a non-circular shape.

## Description

### BACKGROUND OF THE INVENTION

### Field of the Invention

The present invention relates to a valve for a respiratory device, one example of such valves are expiration and inspiration valves. Respiratory devices are, for example, anaesthetic machines, medical ventilators etc. More particularly, the invention relates to a valve device for controlling the flow of at least one fluid through at least one channel. In particular it relates to reduce the flow resistance and the pressure drop through a valve, such as expiration valves or inspiration valves.

### Description of the Prior Art

It is known that when designing low pressure valves, especially in the field of gas control in medical ventilators, is it of high importance that the flow channel in the valve has low flow resistance, low pressure drop and no or little turbulence. Also, it is often desirable for the design of the valve to be small and light.

The most common design of low pressure expiration valves comprises a circular disk lying against the end of a tube forming a valve seat. US patent 5,127,400 discloses an example of such a design. These valves are generally devised as disk valves, i.e. the directional valve has a disk-shaped valve body that rests loosely on a valve seat.

An improved valve with reduced flow resistance and lower pressure drop would be advantageous. It would be further advantageous if the valve had an improved stability thereby reducing the likelihood of the valve starting to oscillate when connecting means with a pressure drop after the valve.

### SUMMARY OF THE INVENTION

Accordingly, embodiments of the present disclosure preferably seek to mitigate, alleviate or eliminate one or more deficiencies, disadvantages or issues in the art, such as the above-identified, singly or in any combination by providing a device, or method according to the appended patent claims for providing an improved valve for a respiratory device, such as an expiration valve or inspiration valve. The valve may be a disk-valve but may equally be other types of valves. Disclosed herein are device and methods for providing the improved valve.

According to one aspect of the disclosure, a valve for a respiratory device is disclosed. The valve includes a valve housing having an inlet and an outlet. The valve further includes a tubular flow channel arranged in the valve housing and thereby forming a chamber between an inner surface of the valve housing and an outer surface of the tubular flow channel. One end of the tubular flow channel is the inlet and an edge at the other end of the tubular flow channel is configured as a valve seat. In this way, a fluid flow from the inlet flows through the tubular flow channel and over the valve seat into the chamber. The outlet has an outlet housing and the outlet is arranged perpendicular to the inlet. The outlet of the valve has a non-circular shape.

In some examples of the valve, the outlet is arranged so that at least a portion of an inner surface of the outlet housing is connected with the inner surface of the valve housing at a first transition portion. The first transition portion is arranged, in relation to the chamber, between a maximum and/or minimum point of the outer surface of the tubular channel and/or a maximum and/or minimum point of the inner surface of the valve housing.

In some examples of the valve, the first transition portion is located inside the chamber so that the inner surface of the outlet extending out from the chamber.

In some examples of the valve, the portion of said inner surface of the outlet housing, connected to the inner surface of the valve housing, is arranged tangential to the chamber.

In some examples of the valve, the portion of the inner surface of the outlet housing, connected to the inner surface of the valve housing, allows a flow of a fluid out from the chamber in a tangent direction along the inner surface of the outlet housing.

In some examples of the valve, an opposite portion to the portion of the inner surface of the outlet housing connected to the inner surface of the valve housing is arranged so that the opposite portion is connected to the inner surface of the valve housing at a second transition portion.

In some examples of the valve, a height of the outlet is about the same as an inner diameter of the valve housing.

In some examples of the valve, one portion of the outlet is planar or straight and arranged parallel to the tubular flow channel.

In some examples of the valve, one portion of the outlet is planar or straight and arranged parallel to the valve seat.

In some examples of the valve, the outlet has a rectangular shape, such as a rectangle with rounded corners.

In some examples of the valve, the outlet has an elliptical shape.

In some examples of the valve, the outlet has a Check valve assembled connected to it.

In some examples of the valve, the valve is an expiration valve or inspiration valve.

According to one aspect of the disclosure, a kit is disclosed. The kit includes a valve according to the disclosure and an adaptor for connecting a tube to a non-circular outlet, such as a rectangular or elliptical outlet.

According to one aspect of the disclosure, a method of reducing flow resistance in a disk-valve for a respiratory device. The method comprises providing a non-circular outlet to the disk-valve. The outlet is arranged perpendicular to an inlet.

In some examples the method includes, arranging the outlet so that at least a portion of an inner surface of an outlet housing is connected with an inner surface of the valve housing at a transition portion. The transition portion is arranged, in relation to the chamber, between a maximum and/or minimum point of the outer surface of the tubular channel and/or a maximum and/or minimum point of the inner surface of the valve housing.

In some examples of the method, the outlet has a rectangular shape.

It should be emphasized that the term "comprises/comprising" when used in this specification is taken to specify the presence of stated features, integers, steps or components but does not preclude the presence or addition of one or more other features, integers, steps, components or groups thereof.

### BRIEF DESCRIPTION OF THE DRAWINGS

These and other aspects, features and advantages of which examples of the disclosure are capable of will be apparent and elucidated from the following description of embodiments of the present disclosure, reference being made to the accompanying drawings, in which
Figs. 1A to 1D are illustrating schematic overviews of an exemplary disk-valve;
Figs. 2A to 2D are illustrating schematic overviews of an exemplary disk-type valve;
Figs. 3A and 3B Figures 3A and 3B are illustrating a schematic example of relation between areas of a round and a rectangular check-valve and the length of the valve housing;
Fig. 4 is illustrating a schematic overview of an exemplary disk-type valve;
Figs. 5A and 5B are illustrating two exemplary valves of the disclosure, Fig. 5A with a circular outlet and Fig. 5B with a rectangular outlet;
Figs. 6A and 6B are illustrating two exemplary valves of the disclosure, Fig. 6A with a circular outlet and Fig. 6B with a rectangular outlet.

### DESCRIPTION OF EMBODIMENTS

Specific examples of the disclosure now will be described with reference to the accompanying drawings. This disclosure may, however, be embodied in many different forms and should not be construed as limited to the examples set forth herein; rather, these examples are provided so that this disclosure will be thorough and complete, and will fully convey the scope of the disclosure to those skilled in the art.

The following description focuses on an example of the present disclosure applicable to a valve for a respiratory device. For example, the valve may be used in devices for patient monitoring, anaesthesia, medical ventilators, etc. However, it will be appreciated that the description is not limited to this application but may be applied to many other systems where a fluid pump is required.

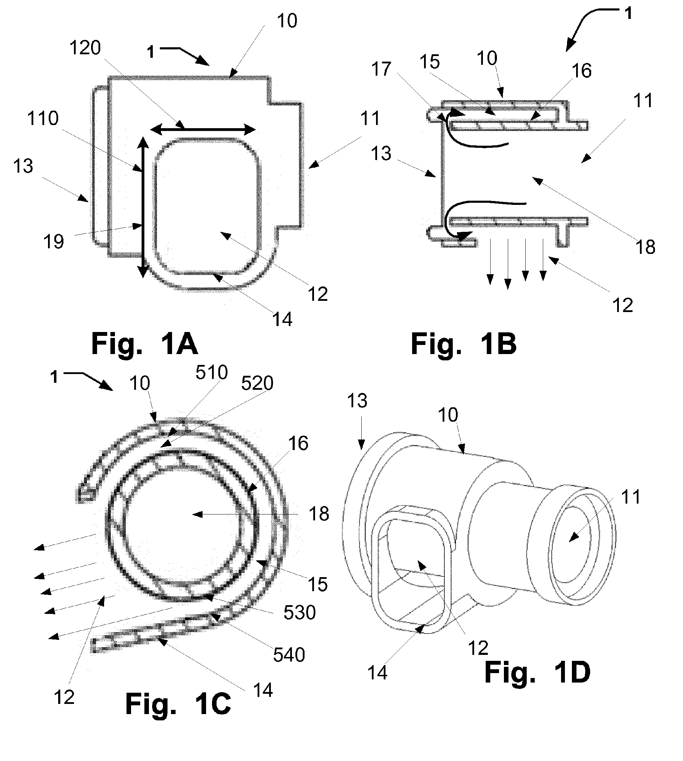

Figure 1A illustrates an exemplary side view of a valve 1. The valve has a valve housing 10 with an outlet 12 and an inlet 11. The outlet has at least one portion 14 that goes all the way out to a chamber edge or close to the chamber edge. In the illustration the outlet 12 is illustrated as rectangular but other shaped may be possible such as the outlet 12 being elliptical.

Figure 1B illustrates an exemplary cross sectional view of the valve 1 in figure 1A. The valve 1 has a valve housing 10. The valve housing 10 may have an inner tubular or cylindrical channel. A tubular flow channel 18 is arranged inside the valve housing so that a space between an inner surface of the valve housing 10 and an outer surface 16 of the tubular flow channel 18 forms a chamber 15.

An inlet 11 is, when in use, in a fluid connection via a conduit or a tube to a patient connected to a medical ventilator or anaesthesia machine the valve 1 may be used either as an expiration valve or inspiration valve.

The inlet 11 forms an inlet to the valve 1.

An outlet 12 is arranged at the side of the valve housing 10, perpendicular to the inlet 11.

At an opposite side to the inlet 11 on the tubular flow channel 18, the edges are configured to be a valve seat 17. A valve body 13 rests at the valve seat 17 when the valve is closed. When the valve is open there is a gap between the valve body 13 and the valve seat 17 allowing fluid to flow from the tubular flow channel 18 into the chamber 15 and out through the outlet 12. In this example is the valve body 13 illustrated as disk-shaped, but other designs are possible. The surface of the valve body 13 in contact with the valve seat 17 should be made of a soft material.

Some improvements are achieved by arranging at least one portion 14 of the outlet housing to go all the way out to a chamber edge or close to a chamber edge is lower pressure drop and lower flow resistance than for a standard arrangement of an outlet for a disk- type valve. Also, by having a non-circular outlet, such as a rectangular or elliptic, the cross-sectional flow area of the chamber 15 may be reduced in size compared to a normal disk-valve with a circular outlet as the outlet may be designed with a larger area increasing the flow out from the chamber. A smaller cross-sectional flow area of the chamber 15 increases the stability of the valve. Problems with instability may lead to oscillation of the valve if means with a pressure drop is connected to the outlet.

Stability is particularly important if such means with a pressure drop may for example be flow measuring means, a check-valve or an evacuation apparatus or tube for gases.

An advantage with archiving a larger cross-sectional flow area of the outlet is that a check-valve connected to the outlet will have the same larger area. Hence pressure drop of the check-valve may also be reduced.

Figure 1C is illustrating a schematic cross-sectional view of the valve 1. As illustrated in this example of a disk-valve, a valve housing 10 may have an inner tubular or cylindrical channel. A tubular flow channel 18 is arranged inside the valve housing 10 so that a space between an inner surface of the valve housing 10 and an outer surface 16 of the tubular flow channel 18 forms a chamber 15. The valve housing 10 has an outlet 12. The outlet has at least one portion 14 that goes all the way out to a chamber edge or close to the chamber edge.

By arranging the at least one portion 14 of the outlet to go all the way out to a chamber edge or close to the chamber edge, the portion 14 may be arranged so that at least a portion of an inner surface of the outlet housing is connected with the inner surface of the valve housing at a first transition portion. The first transition portion may be arranged, in relation to the chamber 15, between a maximum point 520 of the outer surface of the tubular channel and a maximum point 510 of the inner surface of the valve housing. Alternatively and/or additionally, the first transition portion may be arranged, in relation to the chamber 15, between a minimum point 530 of the outer surface of the tubular channel and a minimum point 540 of the inner surface of the valve housing.

Additionally and/or alternatively, in some examples, by arranging the at least one portion 14 of the outlet to go all the way out to a chamber edge or close to the chamber edge, the transition portion is located inside the chamber 15 so that a portion of an inner surface of the outlet housing may extend out from the chamber 15. In this way at least a portion of the inner surface of the outlet housing extends into the chamber 15 forming a part of the inner surface of the valve housing.

Additionally and or alternatively, by arranging the at least one portion 14 of the outlet housing to go all the way out to a chamber edge or close to the chamber edge, the at least one portion of the inner surface of the outlet housing may be connected to the inner surface of the valve housing so that the at least one portion 14 of the inner surface of the outlet housing may be arranged tangential to the chamber 15, as illustrated in the example in figure 1C.

Figure 1D is illustrating a three-dimensional view over the exemplary disk-type valve illustrated in figures 1A to 1C.

By arranging the at least one portion 14 of an inner surface of the outlet housing as described herein in relation to Figure 1A to 1D, may allow a fluid to flow out from the chamber 15 in a tangent direction along the inner surface of the outlet housing.

To further improve the flow out from the chamber 15 and thereby reduce the flow resistance and the pressure drop of the valve 1, one portion of the outlet 12 may be planar and parallel to the tubular flow channel. The reduced flow resistance and pressure drop is further enhanced if the planar portion of the outlet 12 is the same as the portion 14 which is connected to the inner surface of the valve housing 10.

The planar side which is parallel to the tubular flow channel 18 may create a straight flow path out of the chamber 15. Additionally, a wider 120 planar or straight side of the outlet 12 creates a larger cross-sectional flow area along the planar or straight side in direct fluid communication with the chamber 15 compared to a circular outlet. Hence the flow out from the chamber 15 may increase and have a more homogenous velocity profile of the flow compared to a circular outlet. Additionally, due to the improved flow out from the chamber 15, the cross-sectional flow area of the chamber 15 may be made smaller than for a conventional disk-valve with a circular outlet. A smaller cross-sectional flow area of the chamber 15 will improve the stability of the valve 1. Hence the valve will less likely start to oscillate.

Additionally and/or alternatively, in some example of the valve 1, to improve the flow out from the chamber 15 and thereby reduce the flow resistance and the pressure drop of the valve 1, one portion of the outlet 12 may be planar and parallel to the valve seat 17.

The planar side 19, which is parallel to the valve seat 17, may create a straight flow path out of the chamber 15. Additionally, a longer height 110 of the planar or straight portion 19 will further improve the fluid flow out of the chamber 15, hence reduce the resistance. The highest improvement is archived if the planar or straight portion 19 of the outlet 12, parallel to the valve seat 17, is the side closest to the valve seat 17.

Additionally, a large outlet area lowers the pressure drop. When having a rectangular shaped outlet area, a large area of the outlet may effectively be created compared to a circular outlet. Thus the flow out from the chamber 15 may increase and have a more homogenous velocity profile of the flow compared to a circular outlet.

Additionally, due to the improved flow out from the chamber 15, the cross-sectional flow area of the chamber 15 may be made smaller than for a conventional disk-valve with a circular outlet. A smaller cross-sectional flow area of the chamber 15 will improve the stability of the valve 1. Hence the valve will less likely start to oscillate.

Additionally, in some examples of the disclosure, the outlet 12 may be rectangular. A rectangular outlet 12, as illustrated in the figures, may further enhance the flow out from the chamber 15 compared to only having one side that is planar or straight as the cross-sectional flow area can be made even larger. Hence the pressure drop and the flow resistance may be further reduced. A rectangular outlet 12 will also have an even more homogenous velocity profile of the flow compared to if only one side is planar or straight. Also due to the improved flow out from the chamber 15 with a rectangular outlet 12, the cross-sectional flow area of the chamber 15 may be even further reduced compared to when only one side is planar or straight.

Additionally, by using a rectangularly shaped outlet 12,the outlet may be optimized so that the sides of the rectangular outlet is as far as way as possible from the center of the outlet 12, which may improve the homogeneity of the fluid flow out from the outlet 12.

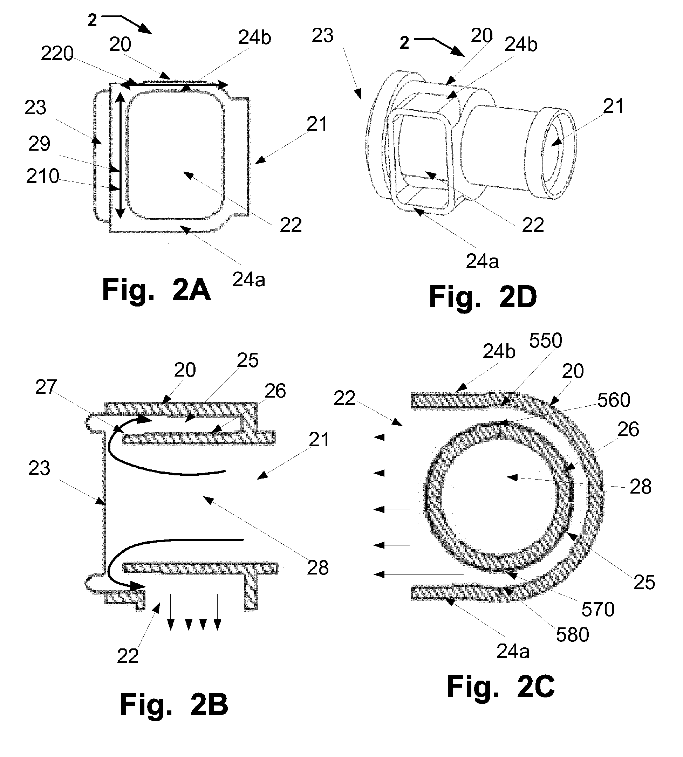

Figures 2A to 2D are illustrating an exemplary valve 2 according to the principles disclosed in connection to figure 1A to 1D. The valve 2 has a valve housing 20 with an outlet 22 and an inlet 21. The outlet has two portions 24a and 24b that goes all the way to the chamber edges or close to the chamber edges. The outlet 22 is arranged so that a portion 24a of an inner surface of outlet housing is connected with the inner surface of the valve housing 20 at a first transition portion which may be arranged, in relation to the chamber 25, between a minimum point 570 of the outer surface of the tubular channel and a minimum point 580 of the inner surface of the valve housing. Additionally, the outlet 22 is arranged so that a portion 24b of an inner surface of outlet housing is connected with the inner surface of the valve housing 20 at a second transition portion which may be arranged, in relation to the chamber 25, between a maximum point 560 of the outer surface of the tubular channel and a maximum point 550 of the inner surface of the valve housing.

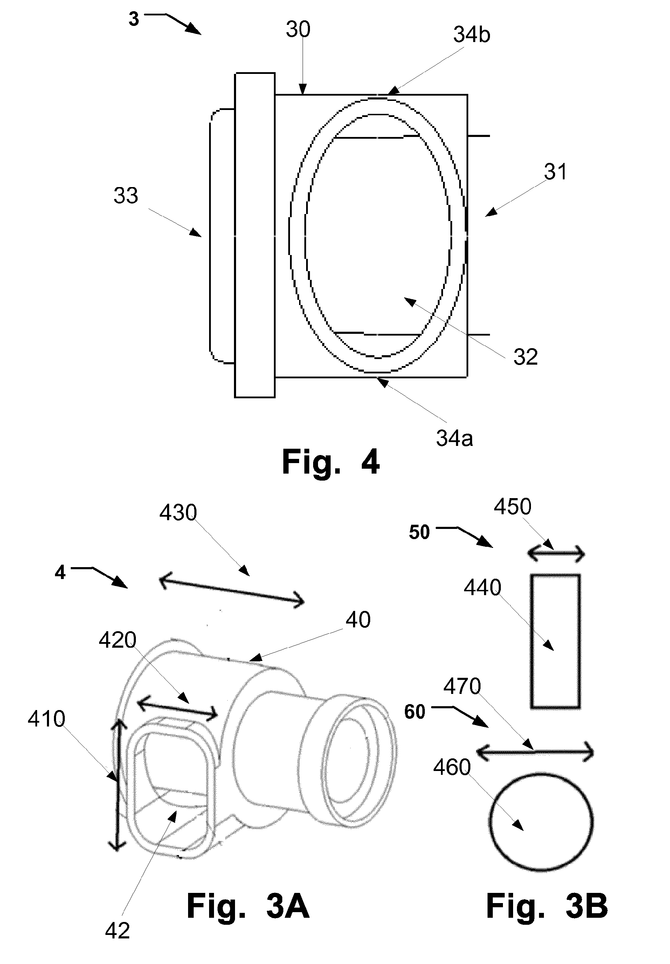

In the illustration the outlet 22 is illustrated as rectangular but other shaped may be possible such as the outlet 22 being elliptical, as illustrated in figure 4.

The valve housing 20 may have an inner tubular or cylindrical channel. A tubular flow channel 28 is arranged inside the valve housing 20 so that a space between an inner surface of the valve housing 20 and an outer surface 26 of the tubular flow channel 28 forms a chamber 25.

An inlet 21 is, when the valve 2 is in use, in a fluid connection via a conduit or a tube to a patient connected to a medical ventilator or anaesthesia machine.

An outlet 22 is arranged at the side of the valve housing 20, perpendicular to the inlet 21.

On the tubular flow channel 28 at an opposite side to the inlet 21 are the edges configured to be a valve seat 27. A valve body 23 rests at the valve seat 27 when the valve is closed. When the valve is open there is a gap between the valve body 23 and the valve seat 27 allowing fluid to flow from the tubular flow channel 28 into the chamber 25 and out through the outlet 22. In this example is the valve body 13 illustrated as disk-shaped, but other designs are possible. The surface of the valve body 23 in contact with the valve seat 27 should be made of a soft material.

The height 210 of the outlet 22 may be about the same size as the outer size of the chamber 25, i.e. as the inner diameter of the valve housing 20. A large height 210 may improve the flow out from the chamber 25 as there are two portions 24a and 24b that are connected to the chamber 25 as disclosed in herein in connection to the portion 14 of figures 1A to 1D. Hence the flow characteristics may be further improved.

As disclosed in relation to figures 1A to 1D having one planar or straight side parallel to the tubular flow channel 28 on the outlet improves the flow characteristics out from the chamber 25. Also, a wider 220 planar or straight portion parallel to the tubular flow channel, may further improve the fluid flow out of the chamber.

Additionally and/or alternatively, in some examples of the valve 2, by arranging a planar or straight portion 29 of the outlet parallel to the valve seat 27 may improve the flow characteristics out from the chamber 25, as disclosed in relation to figures 1A to 1D. The best improvement over a circular outlet is archived if the planar or straight portion 29 of the outlet 22, parallel to the valve seat 27, is the side closest to the valve seat 27. By using a rectangular outlet 22, as illustrated in figures 2A to 2D, two straight flow paths may be created out from the chamber 25. As both these planar or straight portions 24a and 24b are connected to the chamber 25, the flow resistance of the valve 2 is further reduced. The large cross-sectional flow area of such a rectangular outlet 22 lowers the pressure drop.

Additionally, when having a rectangular shaped outlet 22 with two portions 24a and 24b that are connected to the chamber 25, a large cross sectional flow area may effectively be created hence the homogenousity of the flow velocity may be further improved.

Additionally, due to the large cross-sectional flow area, the stability of the valve 2 may be further enhanced as the cross-sectional flow area of the chamber 25 may be further reduced.

If a round tube needs to be connected to the outlet of the valves disclosed in connection to figures 1A to 1D and figures 2A to 2D. An adapter with a rectangular shaped inlet and a round shaped outlet can be used. Alternatively if the tube or conduit is flexible it may be forced over the non-circular outlet. A round tube or a conduit connected to the non-circular outlet will have little or non-effect on the improved performance of the valve.

A check valve may be assembled with a screw outside the flow channel of the outlet. Alternatively, the check-valve may be assembled against the valve housing with an integrated molded shaft with a knob. In either cases there may be nothing obstructing the flow.

Figures 3A and 3B are illustrating a schematic example of relation between areas of a round and a rectangular check-valve and the length of the valve housing. In figure 3A is a schematic disk-valve 4 illustrated. 410 is the height of the outlet 42, 420 is the width of the outlet 42 and 430 is the length of the valve housing 40.

Figure 3B is illustrating two schematic check-valves, one rectangular check-valve 50 matching the height 410 and the width 420 of the disk-valve 4 and one circular check-valve 60.

The pressure drop of a check-valve has a negative effect on the disk-valve 4. With a rectangular shape of the check-valve as in figure 3B, the effect may be less compared to a circular check-valve. The reason is that the area 440 of a rectangular check-valve 50 may be made larger than the area 460 of a circular check-valve 60 when the width 450 of the rectangular check-valve 50 is the same as the diameter 470 of a circular check-valve 60. A larger area of the check-valve will reduce the pressure-drop of the check-valve.

The disk-valve disclosed herein is more stable than a disk-valve reducing the risk of the valve to oscillate when used with a check-valve. This risk may be reduced further by using a rectangular check-valve with a larger area matching the area of the outlet of the disk-valve.

Another advantage of using a rectangular outlet and check-valve is that for a specific outlet area, such as the area 440 of the rectangular check-valve 50 is the same as the area 460 of the circular disk-valve 60. The length 430 of the valve housing 40 may be made shorter than if using a circular outlet as the width 450 may be shorter than the diameter 470.

The valve 4 may still have a reduced risk for oscillating due to the stability given by the rectangular outlet 42.

Additionally, the same applies when using an elliptically shaped outlet and check-valve.

Figure 4 is illustrating an example of a disk-valve 3 having a valve housing 30, and inlet 31 and a disk shaped A valve body 33 which rests on a valve seat (not shown) when the valve is closed. The disk-valve 3 has an elliptic outlet 32 where the outlet housing goes from one edge 34a of the chamber to another edge 34b of the chamber.

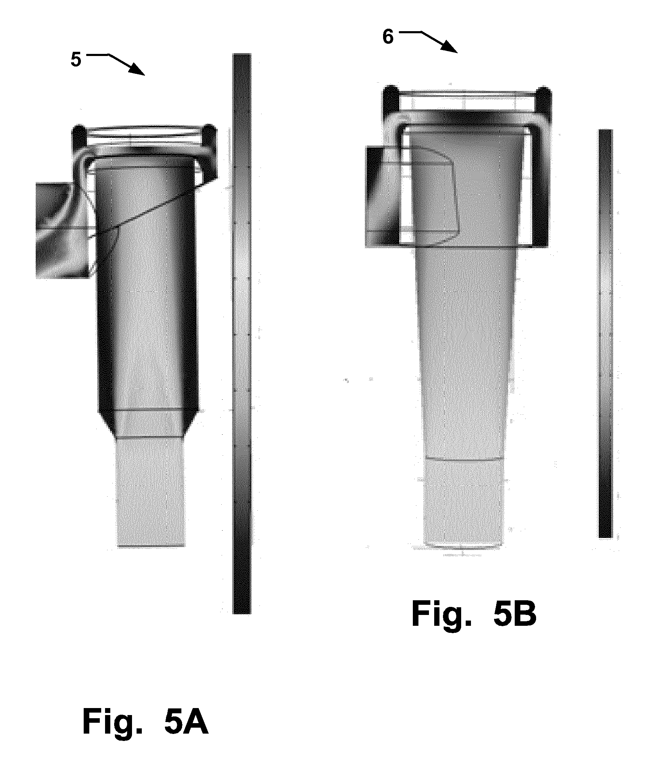

Figure 5A and 5B illustrate two valves. Figure 5A illustrates a valve with a circular outlet and figure 5B illustrates a valve with a rectangular outlet. For these simulations the velocity was 30 l/min and the opening valve gap 2.5 mm. As seen, the circular outlet model has a larger velocity concentrated to the lower part of the outlet tube while the rectangular outlet model has a more homogeneous outlet flow. The flow at the valve seat is concentrated at the closest way to the outlet for the circular outlet model. But for the rectangular outlet model the flow is spread out all around the valve seat. The more homogenous velocity experienced in figure 5B is due to the rectangular shape combined with the large outlet that goes all the way out to the chamber edges.

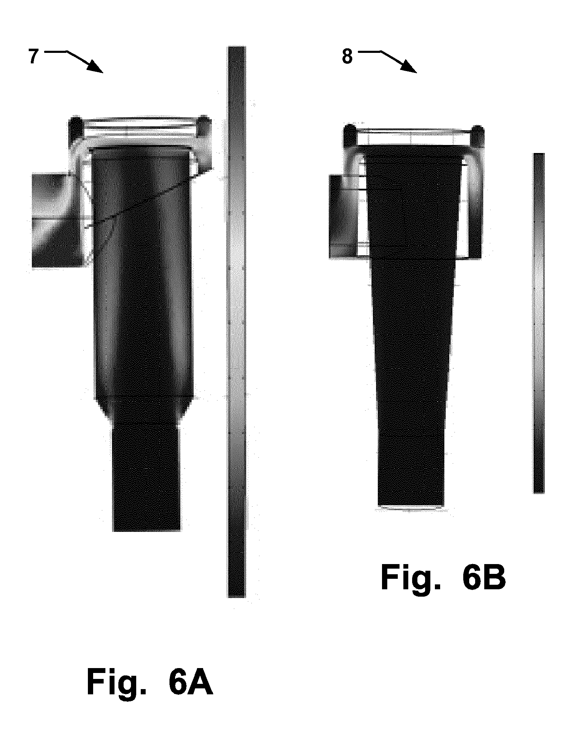

Figure 6A and 6B illustrate two valves. Figure 6A illustrates a valve with a circular outlet and figure 6B illustrates a valve with a rectangular outlet. For these simulations the velocity was 30 l/min and the opening valve gap 2.5 mm.

Figure 6A and 6B illustrates the turbulent kinetic energy. The rectangular shaped model has a much lower turbulence level at the outlet and at the valve seat. A low turbulence at the outlet can be good if you for example want to attach a flow sensor after the valve outlet. A homogenous turbulence around the valve seat is good for the stability of the valve.

| | Rectangular outlet | Circular |
|---|---|---|
| Lit/min | Δp (mBar) | Δp (mBar) |
| 30 | 0.14 | 0.3 |
| 60 | 0.53 | |
| 150 | 3.3 | 6.5 |
| 180 | 4.7 | |

The table above show simulated pressure drops of a rectangular shaped and a circular shaped outlet at different flows. Tests have showed that the simulated pressure drop is very close to real measured values.

While several examples of the present disclosure have been described and illustrated herein, those of ordinary skill in the art will readily envision a variety of other means and/or structures for performing the functions and/or obtaining the results and/or one or more of the advantages described herein, and each of such variations and/or modifications is deemed to be within the scope of the present disclosure. More generally, those skilled in the art will readily appreciate that all parameters, dimensions, materials, and configurations described herein are meant to be exemplary and that the actual parameters, dimensions, materials, and/or configurations will depend upon the specific application or applications for which the teachings of the present disclosure is/are used. Also, different method steps than those described above, performing the method by hardware, may be provided within the scope of the disclosure. The different features and steps of the disclosure may be combined in other combinations than those described. The scope of the invention is only limited by the appended patent claims.

## Claims

1. A valve for a respiratory device, comprising a valve housing having an inlet and an outlet; a tubular flow channel arranged in said valve housing thereby forming a chamber between an inner surface of said valve housing and an outer surface of said tubular flow channel, one end of said tubular flow channel is said inlet and an edge at the other end of said tubular flow channel is configured as a valve seat so that a fluid flow from said inlet flows through said tubular flow channel and over said valve seat into said chamber, and wherein said outlet has an outlet housing and said outlet is arranged perpendicular to said inlet, and wherein said outlet has a non-circular shape.

2. The valve according to claim 1, wherein said outlet is arranged so that at least a portion of an inner surface of said outlet housing is connected with said inner surface of said valve housing at a first transition portion, and wherein said first transition portion is arranged, in relation to said chamber, between a maximum and/or minimum point of said outer surface of said tubular channel and/or a maximum and/or minimum point of said inner surface of said valve housing.

3. The valve according to claim 1, wherein said first transition portion is located inside said chamber so that said inner surface of said outlet extending out from said chamber.

4. The valve according to any of claims 2 or 3, wherein said portion of said inner surface of said outlet housing, connected to said inner surface of said valve housing, is arranged tangential to said chamber.

5. The valve according to any of claims 2 to 4, wherein said portion of said inner surface of said outlet housing, connected to said inner surface of said valve housing, allows a flow of a fluid out from said chamber in a tangent direction along said inner surface of said outlet housing.

6. The valve according to any of claims 2 to 5, wherein an opposite portion to said portion of said inner surface of said outlet housing connected to said inner surface of said valve housing is arranged so that said opposite portion is connected with said inner surface of said valve housing at a second transition portion, and wherein said second transition portion is arranged between a maximum and/or minimum point of said outer surface of said tubular channel and/or a maximum and/or minimum point of said inner surface of said valve housing.

7. The valve according to claim 6, wherein a height of said outlet is about the same as an inner diameter of said valve housing.

8. The valve according to any of claims 1 to 7, wherein one portion of said outlet is planar or straight and arranged parallel to said tubular flow channel.

9. The valve according to any of claims 1 to 8, wherein one portion of said outlet is planar or straight and arranged parallel to said valve seat.

10. A valve according to any of claims 1 to 9, wherein said outlet has a rectangular shape, such as a rectangle with rounded corners.

11. A valve according to any of claims 1 to 8, wherein said outlet has an elliptical shape.

12. The valve according to any of claims 1 to 11, wherein said outlet has a Check valve assembled connected to it.

13. The valve according to any of claims 1 to 12, wherein said valve is an expiration valve or inspiration valve.

14. A kit comprising, a valve according to any of claims 1 to 13 and an adaptor for connecting a tube to a non-circular outlet, such as a rectangular or elliptical outlet.

15. A method of reducing flow resistance in a disk-valve for a respiratory device, comprising providing a non-circular outlet to said disk-valve, wherein said outlet is arranged perpendicular to an inlet.

16. The method according to claim 15, arranging said outlet so that at least a portion of an inner surface of an outlet housing is connected with an inner surface of said valve housing at a first transition portion, and wherein said first transition portion is arranged, in relation to said chamber, between a maximum and/or minimum point of said outer surface of said tubular channel and/or a maximum and/or minimum point of said inner surface of said valve housing.

17. The method according to any of claims 15 or 16, wherein said outlet has a rectangular shape.
